# EUROPEAN PATENT APPLICATION

(11) **EP 1 027 874 A2**
(43) Date of publication of application: **16.08.2000**
(21) Application number: 00102625.1
(22) Date of filing: 08.02.2000
(51) Int. Cl.: A61F 13/496

(54) **Disposable absorbent pull-up type diapers and incontinent briefs**

(30) Priority: 10.02.1999 US 247629
(71) Applicant: First Quality Enterprises, Inc., McElhattan, Pennsylvania 17748 (US)
(72) Inventor: Karami, Hamzeh, McElhattan, PA 17748 (US)
(74) Representative: Motsch, Andreas

(57) **Abstract**

An integral disposable pull-up absorbent article is provided having elasticated front and back waist portions, an insert member having an absorbent core comprising an elasticated crotch region, and at least one elastic member disposed in the longitudinal direction on each side in the crotch region. The pull-up article also has a pair of leg openings each having a leg cuff comprising a thigh elastic member which does not intersect any of the crotch elastic members.

Also provided are disposable pants and briefs of the general structure as the pull-up article described herein, but without an absorbent core member.

## Description

### FIELD OF THE INVENTION

The present invention relates generally to disposable absorbent articles such as pants and briefs, and is more particularly related to disposable absorbent pants, diapers and adult incontinent briefs of the so-called "pull-ups" or "pull-on" variety. In one particular aspect, this invention relates to such pull-on pants, diapers and adult incontinent briefs which, due to their unique construction and configuration, provide improved body fitness and comfort to the wearer while affording excellent protection against leakage of urine and other body exudates.

### BACKGROUND OF THE INVENTION

Disposable absorbent articles such as disposable baby diapers and adult incontinent briefs, underpants, guards and the like articles are widely used in the homes and in various health care facilities and institutions. Indeed the use of such articles has become a sanitary practice, and while initially such absorbent articles were used mostly for baby care, more recently their use has been expanded for adults as well. In both instances, the absorbent article must be designed to effectively prevent leakage of urine and other fecal materials, while insuring body fit and comfort.

Present commercially available absorbent articles are generally unitary in structure, pre-shaped and pre-folded, and comprise an absorptive pad having a liquid permeable top sheet facing the wearer's body, a liquid impermeable backsheet on the opposite side, and an absorbent sheet or panel disposed between the top sheet and the back sheet. The absorbent article comprises a front side portion, a crotch portion and a backside portion, and further includes elastic members along the circumference of the waist and around the leg openings. While the heretofore commercially available absorbent articles have been somewhat effective against leakage of body fluids and fecal materials, and have therefore met some degree of acceptability, they have not been entirely satisfactory for their intended applications. In other words, they have not proven to be entirely leak proof, nor have they completely prevented issuance of the body exudates outside the diaper or the underpants. These deficiencies are primarily due to inadequate and loose body fit, which results in leakage of the body fluids and solids through the legs' openings. These problems are even more pronounced in case of adults because of their diverse body shapes and varying contours. Another disadvantage of the commercially available absorbent articles such as diapers, incontinent briefs and the like, is associated with the ability of opening and removing the soiled article without soiling the wearer's legs or body.

There is a plethora of patents which disclose the different attempts made by the prior art workers over the years to eliminate, or at least minimize, the shortcomings of the present commercially available absorbent articles.

United States Patent No. 5,607,416 issued to Masamitsu Yamamoto et al. on March 4, 1997 describes a disposable absorbent pad comprising a pad member adapted to be formed into a boat shape under the contractible forces of elastic members contained side flaps, and an elastic support member; longitudinally opposite sides of the pad member being connected to the front and rear sides of the support member by end flaps. Each of the end flaps comprises a top sheet and a back sheet and is divided into a pair of end flap halves by a slit so as to function as a suspending strap. The end flaps halves are set apart in a V-shape with the slit there between as the support members is stretched and contributes to suspend the pad member with high stability. The absorbent pad described in the Yamamoto et al. patent does not provide for forces to counteract the weight of the soiled pad which eventually stretches the crotch region and thus may cause leakage of urine and other body exudates through the leg openings.

United States Patent No. 5,204,997 issued to Migakum Suzuki et al. on April 27, 1993 describes a disposable pant-type garment, such as a diaper, which is constructed by attaching elastic surrounding flaps around the leg openings and the waist opening. This garment is not elastically integral between the crotch and the waist portions and is not adjustable around the waist to conformably fit the body shape. Nor does this garment allow adjustment or refastening of the elastic flaps to insure body fit when the garment is soiled.

European patent application 799,002 published June 6, 1996 (WO 96/19169) describes an absorbent article such as a diaper which is provided with a closure system for anchoring the absorbent article to the wearer. The absorbent article is provided with tape tabs which are disposed at an angle of approximately 45 degree relative to the longitudinal side edges. The closure system is designed so that a plane of tension is formed at least about the front waist portion of the article to preclude rollover of the front waist portion.

European patent application 802,778 published July 18, 1996 (WO 96/21412) describes a disposable diaper having an absorbent part, a pair of ear parts projecting in opposite directions from opposite side edges of one of the longitudinal end portions of the absorbent part, and two fastening means are attached to the side edges of the ear parts, respectively. Each fastening means is attached to the ear part in a pulling section of the side edge, overlapping at least part of a first edge section and part of a second edge section so that component tensile forces of a tensile force are applied to the fastening means are distributed at a desired distribution ratio to the waist lapping portion and the leg lapping portion of the absorbent part.

European patent application 814,740 published September 26, 1996 (WO96/29038) discloses a disposable diaper having a pair of ears with oblique side edges which are inclined to extend at a predetermined angle to the longitudinal center axis of the absorbent article. The fastening means are attached to the oblique side edges and oblique side edge has a first oblique side edge and a second the oblique side edge section. Each ear has a stress relaxing structure and each fastening means is attached to the ear in order to distribute the tensile forces.

Other prior art workers have described so called "pull-on garments" in order to overcome the deficiencies and problems associated with the prior type diaper constructions. These patents include United States patent numbers 3,237,625; 3,424,162; 3,488,778; 3,530,859; 3,599,638 3,599,640; 3,613,689; 3,636,953; 3,663,962; 4,205,679; and 4,302,853. The diapers and briefs described in these patents, however, have a common structural deficiency in that they are provided with side seams which are welded together by heat and pressure or vibration (ultrasonic welding). It is a matter of common experience that it has been difficult to achieve a perfect side seal, thus resulting in premature tearing and product malfunction, hence, consumers dissatisfaction. The application of heat and pressure also results in stiffness of side seams which causes discomfort to the wearers. Moreover, application of heat and pressure is incompatible with high-speed productions of the garment.

More recently, United States Patent No. 5,769,838 issued to Kenneth B. Buell et al. on June 23, 1998 describes a pull-on garment provided with a continuous belt in the front region and the back region to distribute the forces generated during use in order to better fit the pull-on garment wearer. As in the above mentioned patent to Douglas, Jr., the article described in Buell et al. does not provide for examination of the condition of the diaper and requires tearing a side seam for its removal, thus causing soiling to the wearer and the applier of the garment.

The foregoing patents by no means constitute an exhaustive list of the patents which reflect the efforts of the prior art workers in this field, but are merely illustrative and are described briefly herein for background purposes. As it can be appreciated, however, notwithstanding attempts by others to provide satisfactory absorbent articles for infants as well as for incontinent adults, there is still a need for providing articles commercially, which are highly effective in preventing leakage of urine and other bodily exudates, and which are comfortable to wear and conformably fit the body contours so as to insure against such leakage and prevent soiling the wearer's body as well as the person who applies the garment to the wearer.

Accordingly, it is an object of the present invention to provide a pull-up type disposable absorbent such as baby diapers, adult incontinent underpants, briefs, guards and the like articles, which overcome the deficiencies and shortcomings of the prior art absorbent articles, including the present commercially available garments used for this purpose.

It is another object of this invention to provide such disposable absorbent articles which, due to their unique construction, provide improved fit to the body and prevent leakage of urine and other body exudates through the leg openings.

It is a further object of this invention to provide pull-up diapers, and briefs of all sizes, which have uniquely elasticated waist, legs and crotch regions in order to ensure against leakage of body fluids and exudates while assuring a more perfect fit to the body of the wearer.

The foregoing and other objects and features of the present invention will be more fully comprehended and appreciated from the ensuing detailed description and the drawing which form parts of the application.

It must be understood throughout this application that the term "pull-up" as used herein is synonymous with "pull-on" as used in some of the prior art patents, including, for example, the aforementioned Buell et al.'s United States Patent No. 5,769,838.

### BRIEF DESCRIPTION OF THE DRAWINGS

In the drawings, wherein like reference numerals are employed to designate like parts:
Figure 1 is a perspective view of the disposable absorbent article of the present invention shown as a pull-up diaper;
Figure 2 is a stretched plan view of the pull-up diaper shown in Figure 1;
Figure 2A is similar to Figure 2 except that the thigh elastic is substantially straight rather than curved as in Figure 2;
Figure 2B is similar to Figure 2A except that the leg openings have a generally rectangular pattern;
Figure 2C is similar to Figure 2 except that the front and back waist portions of the pull-up diaper include Velcro regions adapted to seal the diaper.
Figure 3 is a sectional view taken along the line 3-3 of Figure 2 featuring the various layers of the absorbent article; and
Figure 4 is a sectional view taken along the line 4-4 of Figure 2.

### DETAILED DESCRIPTION OF THE INVENTION

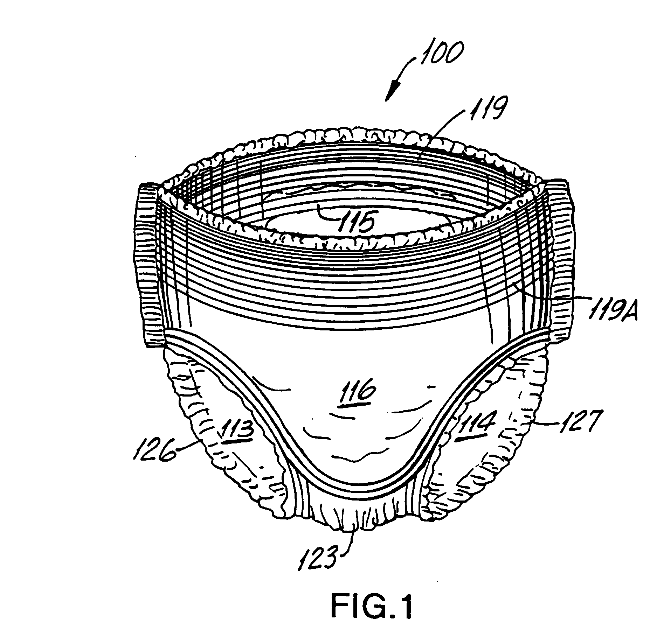

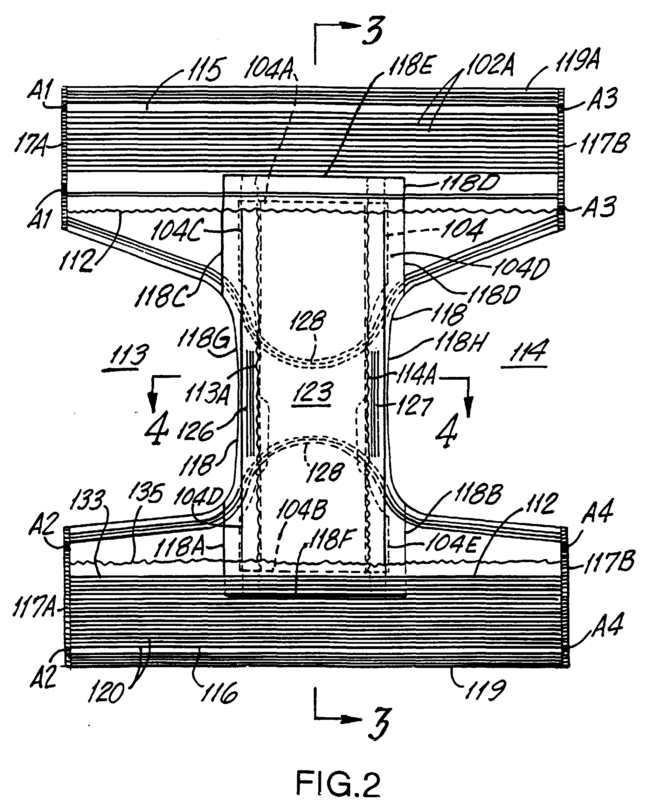

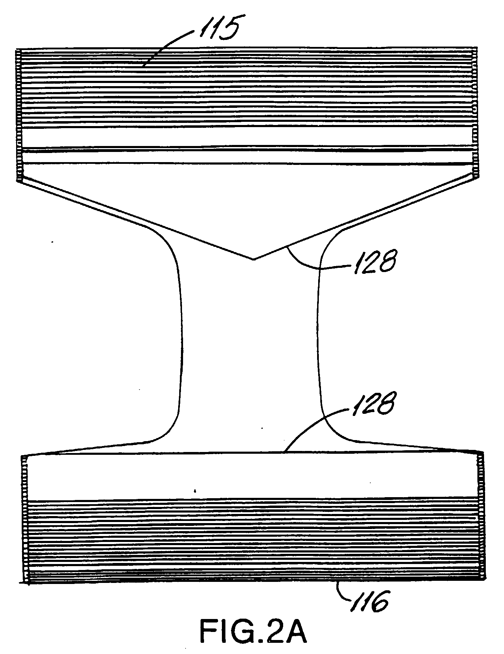

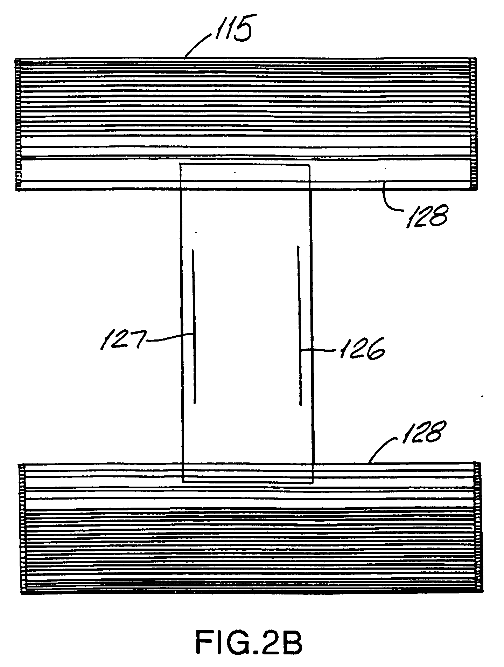

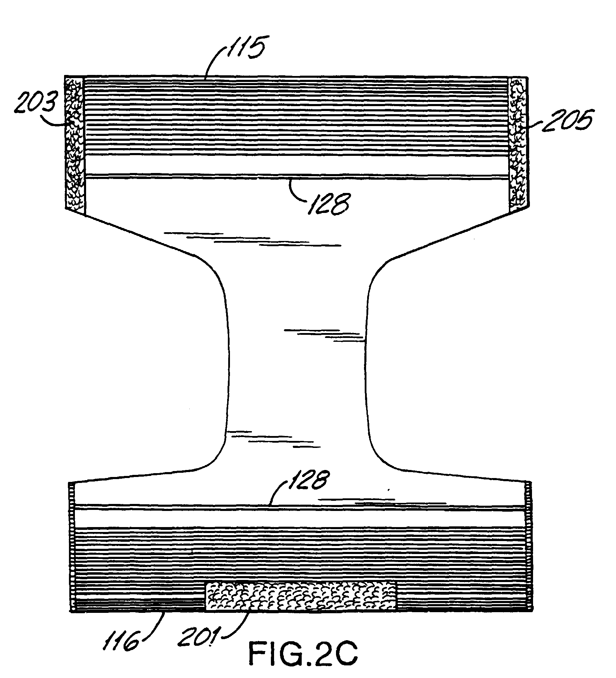

Referring to Figure 1, there is shown a pull-up diaper generally designated as 100 comprising an elasticated back waist portion 115 and an elasticated front waist portion 116. The elasticated back waist portion 115 includes an elastic band comprising a plurality of relatively narrow members 119 spanned across the back waist portion 115, and the elasticated back waist portion 115 also includes a band comprising a plurality of relatively wider elastic members 120 spanned across the back waist portion 115. Similarly, the elasticated front waist portion 116 includes an elastic band comprising a plurality of relatively narrow elastic members 119A spanned across the front waist portion 116, and the elasticated front waist portion 116 also includes an elastic band comprising a plurality of relatively wider elastic members 120A spanned across the front waist portion 116. The diaper 100 also comprises a pair of elasticated leg openings, i.e., a right elasticated leg opening 113 and a left elasticated leg opening 114. The right leg opening 113 is provided at its peripheral edges with the crotch elastic member 126 and the left leg opening 114 is also provided at its peripheral edges with the crotch elastic member 127 each leg opening also comprises a thigh elastic member 128 which is usually tensioned from about 0 to about 400 percent elongation, preferably from about 150 to about 250 percent elongation. The peripheral elastic members 126 and 127 are tensioned between from about 0 to about 400 percent elongation, preferably from about 200 to about 300 percent elongation so that the leg openings fit snugly against the crotch region 123 of the wearer in order to prevent leakage of any urine or other body exudates through the leg openings. When the front and back of the diaper 100 are folded as shown in Figure 1, they can be sealed to form the side seals 117A and 117B thus providing a tight leak proof fit around the waist of the wearer. These side seals may be formed by heat and pressure or, alternatively, they can be formed by a combination of heat and pressure and, in addition, adhesively sealing a finite area on each end of the side seals, such as at A1, A2, A3, and A4, by a hot melt adhesive or cold adhesive material as shown in Figure 2.

Alternatively, as shown in Figure 20, the front and back waist portions may be provided with mechanical sealing means for sealing the diaper. Such means include, as shown in this figure, a male Velcro region 201 in the front waist portion 116, and female Velcro regions 203, 205 in the back waist portion 115, adapted to engage into one another when the front of this diaper is folded onto the back of the diaper, thus providing a leak-proof seal. When Velcro regions are provided as aforesaid, the portions covered by these regions usually need not be elasticated.

Referring again to the drawings, more specifically to Figures 2, 3 and 4, the diaper 100 comprises the diaper insert member 118 which contains the absorbent core 104 sandwiched between the cover or top layer or sheet 101 (facing the body of the wearer) and the polyethylene film 106. The insert 118 is secured, adhesively or by some other suitable means, to a spunbond nonwoven layer 107. Optionally, the absorbent core 104 may be covered by the top and bottom tissue layers 103 and 105, generally made of wood pulp fibers or similar material. An acquisition layer 102 is interposed between the top or cover sheet 101 and the core layer 104 and serves to temporarily retain the body exudates and slowly distribute them through the absorbent core 104 in order to keep the skin dryer. The various layers are generally coextensive with one another and are sealed together to form a sealed composite structure.

As shown in Figure 2, the absorbent core 104 spans part of the length of the diaper 100 terminating at the back edge 104A, the front edge 104B, the right side edges 104C, 104D, and left side edges 104E and 104F. However, as it can also be seen from this figure, the back edge 104A and the front edge 104B of the absorbent core 104 are spaced apart a finite distance, which may be varied, relative to the back and front edges of the diaper. The insert 118 is defined by the longitudinal side edges 118A, 118B, 118C and 118D, the lateral edges 118E and 118F, and includes the necked down region defined by the necked down side edges 118G and 118H. The necked down region defined by the necked down side edges 118G and 118H is elasticated at both sides by the elastic members 126, 127.

As previously mentioned, the leg openings 113, 114 is each is tensioned by a thigh elastic member 128 shown as a curved elastic in Figure 2, but may be straight elastic element as in Figure 2B and Figure 2C or may be as a triangular element as in Figure 2A. The thigh elastic may be tensioned minimally, say, from about 0 to about 400% elongation, preferably from about 150 to about 300 percent elongation for more improved fitness about the legs.

As is further shown in Figure 2, the diaper 100 of the present invention has an elasticized crotch region 123 which is provided with one or more spaced-apart right elastic members 126 disposed interiorly of the leg right opening 113 on the right side edge of the insert 118, and one or more spaced-apart left elastic members 127 disposed interiorly of the leg opening 114 on the left side edge of the insert 118. The crotch region 123 has a crotch cuff 113A disposed on the left side thereof and the crotch cuff 114A disposed on the right side thereof. These crotch cuffs are elasticated, having at least one elastic element which is not tensioned, or is minimally tensioned between about 0 to about 50% elongation.

Alternatively, each cuff elastic may be tensioned differently along its length. Thus, for example, parts of each cuff elastic may be tensioned (elongated) from about 0 to about 10% while other parts may be tensioned to a higher degree, say, up to about 50%.

Positioning the crotch elastic members interiorly relative to the leg openings and the insert 118 insures improved fit in the crotch region. While three elastic members are shown, fewer or more elastic members may be used, if desired.

As shown in Figure 2, the thigh elastics 128 and the crotch elastics 126 on the right sides of the diaper 100 define a protective ring around the left leg opening 113 in a discontinuous pattern. Similarly, on the left side of diaper 100 there is shown a discontinuous ring around the right leg opening 114. This construction assures tight, leak proof fit without pinching the body of the wearer.

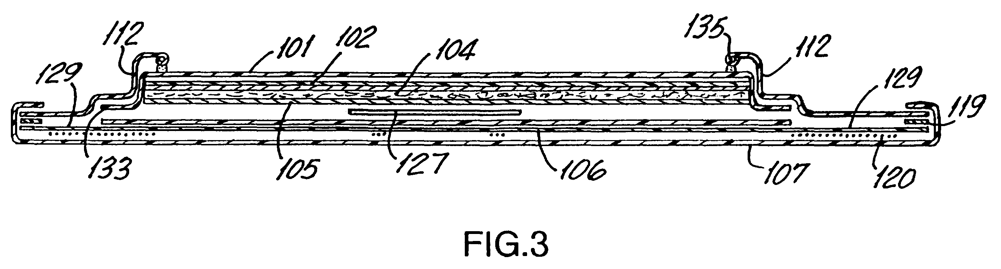

The relatively narrow back and front bands comprising the elastic members 119 and 119A each usually comprises about 3 to about 10 elastic members having a percent elongation of from about 100 to about 600 percent, preferably from about 300 to about 400 percent. The relatively wider back and front bands comprising the elastic members 120 and 120A each usually comprises about 2 to about 16 elastic members and have a percent elongation of from about 100 to about 600 percent, preferably from about 300 to about 400 percent. Also, the elastic members 119 and 120A are wider and spaced closer to one another compared to the spacings of the elastic members 119A and 120. This results in higher retraction forces which help pull the article onto the torso of the wearer without falling down. All the elastic members 119, 119A, 120 and 120A are usually made of synthetic rubber such as Fulfex rubber available from Fulfex Inc., Providence, Rhode Island. Conveniently the elastic members 119, 119A, 120 and 120A are covered by the layer 129 which is made of nonwoven polypropylene as shown in Figure 3.

In addition to the unique arrangements of the elastic members comprised within the relatively narrow and wide band portions as aforesaid, the diaper 100 of the present invention comprises from about 1 to about 6, preferably about 3 crotch elastics 126 and 127 which are spaced apart from each other, located on the right and left of the crotch region 123 on each side of the absorbent core 104, as shown in Figure 2. These crotch elastic members 126, 127 are tensioned and adhesively secured on the sides of the absorbent core but their "active lengths" do not intersect the thigh elastic members 128. The term "active length" refers to the length of the elastic member that is attached under tension interiorly of the insert 118 edges. Extending the crotch elastic members 126, 127 beyond the thigh elastics 128 has no additional advantage since, otherwise, the elastic crotch members may exert unnecessary forces on the pull-on action of the garment and cause skin irritation or redness.

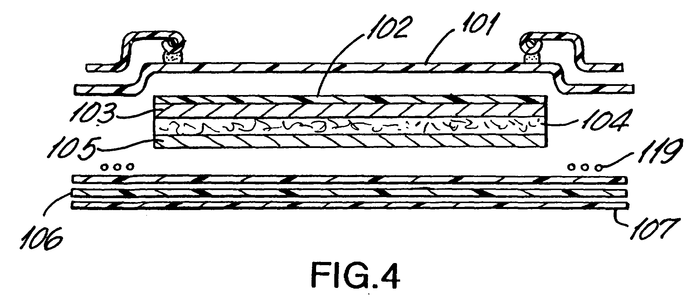

Referring to Figures 3 and 4, the top coversheet or layer 101 is conveniently made of spunbond nonwoven polypropylene which is available from First Quality Fibers, Inc., McEIhattan, PA. The acquisition layer 102 is usually made of chemically bonded nonwoven polypropylene available from American Nonwovens, Columbus, Missouri. Preferably, the width of this layer is substantially the same as the width of the absorbent core 104. This core may be made of wood pulp fibers and superabsorbent polymers such as IM 7000 series available from Clariant Products, Inc., Portsmonth , VA, and Chemdal 200 series, available from Chemdal, Inc., Palantine, Illinois. Alternatively, the absorbent core 104 may be made of dual layer construction, in which case, the absorbent polymer may be securely positioned between each layer of the absorbent material.

The film backing 106 is usually a polyethylene layer which is liquid, air and preferably vapor impermeable, and is placed under the absorbent core member 104 to prevent the body exudates from leaking and otherwise soiling the user's bed and clothing. The width and length of the backing film 106 are generally at least equal to the width and length of the absorbent core 104. Polyethylenes suitable as backing film for the purpose of this invention are available from Clopay Plastics, Cincinnati, Ohio as is further shown in Figures 3 and 4, a layer 107 of spunbond nonwoven polypropylene is disposed as a backing layer and covers the area under the insert 118. This layer is usually coextensive with the overall width and length of the pull-up diaper. This spunbond nonwoven polypropylene is also used to cover the waist elastic members 119, 119A, 120 and 120A.

As is further shown in Figures 2 and 3, there is one elasticated crotch cuff on each side of the garment. Each of these crotch cuffs is formed of a layer of spunbond nonwoven polypropylene laminated by hot-melt adhesive or by heat, and forms a fluid and an air impermeable composite structure. The crotch cuffs are under no tension or are minimally tensioned so that the garment can fit snugly against the body and prevent leakage of body fluids or exudates, without pinching the body of the wearer. Additionally, these cuffs act as barriers against fluid leakage on each side of the absorbent core.

The garment of the present invention also has an elasticated waist cuff 112 which, similar to the crotch cuffs, is not tensioned or is minimally tensioned between about 0 to about 10 percent elongation in order to provide a tight body fit which is leak-proof without pinching the body or causing discomfort to the wearer.

The insert 118 is adhesively secured to the nonwoven backing film 107 and, as shown in Figure 2, the crotch width of the insert 118 is narrower than its width at the waist. As previously mentioned, there are usually three elastic members 126, 127 on each side, although fewer or more elastic members can be used, as desired.

As is further shown in Figure 2, the pull-on diaper of this invention has a waist cuff base 133 with a waist cuff apex 135 on both the front and back of the article. Similar to the crotch cuffs, these waist cuffs prevent fluid leakage from the ends of the core member 104.

While the present invention has herefore been described with certain degrees of particularities and with reference to absorbent diapers and the like garments, it must be understood that several modifications may be made in the structure of the article which are apparent from the foregoing disclosure. For example, the present invention also encompasses articles such as pants and briefs. In such cases, the article need not include an absorbent core as herein described. Nevertheless such articles as pants and non-absorbent briefs are included within the scope of the present invention.

## Claims

1. An integral disposable pull-up absorbent article comprising:
(a) an elasticated waist portion having a front waist portion and a back waist portion,
(b) an insert member comprising an absorbent core having a crotch region, said insert member being defined by opposed generally longitudinal sides and opposed lateral sides, and at least one elastic member in said crotch region disposed in the general longitudinal direction in each of said longitudinal sides, and
(c) a pair of spaced apart leg openings through which extend the legs of the wearer of said article, each of said leg openings having at least one partially elasticiated crotch cuff and at least one thigh elastic member, whereas none of said crotch elastic members intersect any of said thigh elastic members.

2. An integral absorbent pull-up absorbent article as in claim 1 wherein each of said elastic members in said crotch region is disposed interiorly relative to its adjacent leg opening.

3. An integral absorbent pull-up absorbent article as in claim 1 or 2 wherein each of said thigh elastic members is elongated between about 0 and about 400 percent.

4. An integral disposable pull-up absorbent article as in claim 1 wherein said elasticated waist portion comprises an elasticated waist cuff and said elastic member in said waist cuff is elasticated between about 0 and about 10 percent.

5. An integral disposable pull-up absorbent article as in claim 2 or 3 wherein said elasticated waist portion comprises an elasticated waist cuff and said elastic member in said waist cuff is elongated between about 0 and about 10 percent.

6. An integral disposable pull-up absorbent article as in claim 1 wherein each elasticated member in said crotch cuff is at least partially elongated between about 0 to about 50 percent.

7. An integral disposable pull-up absorbent article as in any of the claims 2 to 5 wherein each elasticated member in said crotch cuff is at least partially elongated between about 0 to about 400 percent.

8. An integral pull-up absorbent article as in claim 1 or 3 wherein said elasticated waist portion comprises a portion having relatively narrow elastic members under tension spanning across the waist portion, and a portion having a relatively wider elastic members under tension spanning across the waist portion.

9. An integral pull-up absorbent article as in claim 1 or 3 wherein said elasticated front waist portion and back waist portion each comprises peripheral edges adapted to be sealed together to form a sealed article.

10. An integral absorbent pull-up article as in claim 1 or 3 wherein said waist portion is partially elasticated.

11. An integral absorbent pull-up article as in claim 1 or 3 wherein said crotch elastic members are tensioned to a different degree than the tension in the cuff elastic members.

12. An integral absorbent pull-up article as in claim 8 wherein said front waist portion comprises a region disposed generally in the middle of said waist portion, and said back waist portion comprises a means disposed generally on each lateral side of said back waist portion, said means adapted to inter-engage to provide a leak-proof seal absorbent article.

13. An integral absorbent pull-up article as in claim 12 wherein each of said means comprises a Velcro surface.

14. An integral disposable article comprising
(a) an elasticated waist portion having a front waist portion and a back waist portion
(b) an insert member having an elasticated crotch region, said insert member being defined by opposed generally longitudinal sides, and opposed lateral sides, and at least one elastic member in said crotch region disposed in the general longitudinal direction in each of said longitudinal sides, and
(c) a pair of spaced apart leg openings through which extends the legs of the wearer of said article, each of said leg openings having at least one partially elasticated crotch cuff and at least one elastic member,
wherein none of said crotch elastic members intersect any of said thigh elastic members.
